Europäisches Patentamt

European Patent Office

Office européen des brevets

(11) Publication number: **0 157 024**
**B1**

# EUROPEAN PATENT SPECIFICATION

(45) Date of publication of patent specification: **25.04.90**

(51) Int. Cl.⁵: **A 61 M 1/28**

(21) Application number: **84201886.3**

(22) Date of filing: **17.12.84**

(54) Improved automatic apparatus for peritoneal dialysis.

(30) Priority: **19.12.83 IT 2424783**

(43) Date of publication of application:
**09.10.85 Bulletin 85/41**

(45) Publication of the grant of the patent:
**25.04.90 Bulletin 90/17**

(84) Designated Contracting States:
**AT BE CH DE FR GB IT LI LU NL SE**

(56) References cited:
**EP-A-0 028 371**
**DE-A-1 773 561**
**FR-A-2 486 803**
**US-A-3 709 222**

(73) Proprietor: **SIS TER Spa**
**I-26020 Palazzo Pignano Cremona (IT)**

(72) Inventor: **Gatti, Emanuele**
**Via E. Musa 13**
**I-22100 Como (IT)**
Inventor: **Nardi, Giancarlo**
**Via O. Gentileschi 8**
**I-50100 Pisa (IT)**

(74) Representative: **Dragotti, Gianfranco**
**SAIC BREVETTI s.a.s. Viale Bianca Maria, 15**
**I-20122 Milano (IT)**

Courier Press, Leamington Spa, England.

## Description

The present invention relates to an automatic apparatus for the peritoneal dialysis having simplified structure and improved operation.

When the kidney functionality is seriously compromised, the patient must undergo a dialysis treatment having the purpose of eliminating from the blood of the patient the toxic wastes which are accumulated thereinto and which would quickly and seriously affect the vital functions of the organism. The dialysis consists in the exchange, through a semipermeable membrane, of the toxic substances between the blood and a dialysis solution. This exchange may take place outside the organism, in that case the dialysis being extrabody, and this is the more widespread treatment.

Another type of dialysis which has acquired importance and diffusion in recent years is the peritoneal dialysis, wherein, as the exchange membrane, the peritoneal membrane is used and the dialitic solution is introduced in the peritoneal cavity. It is evident that by this treatment the extrabody circulation of the blood is eliminated with its possible problems and risks, but other problems and drawbacks exist, which are for instance connected to the control of the pressure of the dialysis solution, which fills the peritoneal cavity, the substitution thereof, etc.

DE—A—1773561 discloses an apparatus for the endoperitoneal dialysis wherein a feeding tank is filled, by means of a pump, with fresh dialysing solution, the latter being then conveyed by gravity to the peritoneal cavity during the filling phase of the whole cycle.

The operation of the pump is controlled by valves sensing the level of the dialysis solution within said tank and the corresponding hydraulic load.

US—A—3709222 provides an apparatus for peritoneal dialysis in which a flexible plastic bag is filled with fresh dialysis solution, the latter being in turn fed by gravity to the peritoneal cavity of the patient for the filling thereof.

The fresh solution is restored in the said bag in the same amount by making use of the exhausted dialysis solution discharged from the peritoneal cavity and entering, before the disposal to waste, a two compartment proportioning chamber from which the said flexible bag is fed with the said fresh dialysis solution.

In US—A—4,381,003 an automatic apparatus is described for the peritoneal dialysis. The apparatus disclosed in said US Patent provides for the displacement of the dialysis solution in a closed circuit mainly comprising, besides the necessary ducts, the bags containing the dialysis liquid and the peritoneal cavity of the patient.

The displacement of the liquid is carried out by means of a pump of particular structure having reversible operation.

As above mentioned, the amount of liquid introduced in the peritoneal cavity must be a determined amount, since different amounts might induce damages to the patient (in the case of an excess amount) or an insufficient depuration of blood (lacking amount).

The main problem arising in automatic apparatus for the peritoneal dialysis is thus that of feeding with a predetermined amount of dialysis liquid. There is worth to mention that the containing bags for the collection of the dialysis liquid preferably contain a liquid amount greater than that is normally introduced in the peritoneal cavity with the aim of feeding, at the beginning of each cycle, at least partially fresh dialysis solution.

According to the teaching of said US Patent, the determining of the liquid amount introduced in the peritoneal cavity is carried out through the measurement of the pressure generated by the liquid, and thus by the pump, inside the peritoneal cavity. Such a pressure in the peritoneal cavity is in turn measured, always according to the said US Patent, on the basis of the pressure head of the liquid upstream of the pump (in the direction of filling the peritoneal cavity). More precisely in the duct length between the pump and the peritoneal cavity a shunt is provided, essentially operating as a pressure switch which in turn is adapted to control the operation of the pump, particularly the actuation thereof in the loading or emptying phase with respect to the peritoneal cavity.

One of the problems in the case of the known apparatus for peritoneal dialysis is that of the control of the pump operation and, particularly, of the related control in the emptying phase, when only one flow circuit is used in both directions (namely alternatively for loading and emptying).

In fact it may occur, when the control is carried out by pressure switch sensing means, that the pump continues, to apply a sucking action to the peritonal cavity even if the latter is empty, since the pressure switch is influenced by conditions extraneous to the operation of the dialysis system (fits of coughing, state of agitation of the patient, etc.), thus indicating the existance of a peritoneal pressure and consequently keeping the pump in operation in the emptying phase.

The drawbacks deriving from situations of this kind (first of all the obstruction of the catheter owing to adjacent tissues, which are sucked into the catheter, the lack of inversion of the pump cycle whereby the restarting of the dialitic cycle is delayed and a general sense of discomfort for the patient) are per se evident and don't need further comments.

The purpose of the present invention is that of providing, for the treatments of peritoneal dialysis, an automatic simplified apparatus, which at the same time permits the circulation of the desired amount of dialysis solution between the feeding bags and the peritoneal cavity and of compensating the variations of disturbing pressure acting within the peritoneal cavity, whereby the measurement of the amount of dialysis liquid used is carried out in a manner which is essentially independent from said disturbing conditions.

Automatic apparatus for peritoneal dialysis comprising:

at least one bag (4) for the feeding and collecting of the dialysis solution;

a connecting duct (5) between each bag and the peritoneal cavity of the patient; and

a reversible rotating pump (6) to carry out the circulation of the dialysis solution in said connecting duct towards and away from the peritoneal cavity, characterized by comprising:

first dilatable compensating means (12) having a controlled dilatation and in liquid communication with said peritoneal cavity, said first means (12) with controlled dilatation are connected in series to the length of said connecting duct (5) between the pump (6) and the patient peritoneal cavity and are capable of compensating abrupt pressure variations in said duct the dilatation of said first means being caused by the pressure of the dialysis solution circulating within the same length of duct, and

second means (15, 16, 21, 22, 13) associated to said first means having controlled dilatation capable of measuring their dilatation and correspondingly controlling the pump operation, at least in the phase of filling of the peritoneal cavity with the dialsyis solution.

According to a further feature of the invention said first dilatable means comprise an elastic means generating a force opposing to the dilatation of said bag, said second means consisting of a linear transducer for the measurement of the dilatation of said compensating bag operating between a fixed reference of the dialysis apparatus and a free wall of said dilatable bag.

According to the invention there is thus provided an automatic apparatus for dialysis which is different from those of the same type known to date.

According to a surprising feature of invention there is now made possible the use of only one detecting system which is positioned in only one point of the circuit for the feeding and emptying of the dialysis solution filling the pump on the basis of the preadjustable amount of dialitic solution filling the peritoneal cavity and which contemporarily compensate possible operation errors of the controls owing to disturbing factors (fits of coughing, etc.), with the advantage of using an apparatus simpler with respect to the conventional ones and consequently more reliable, less subjected to risks of contamination of the dialysis solution and lastly more economical.

The presence of dilatable means has also the advantage of damping the pressure pulsations which are generated by the pump, so as to give place to a regular and continuous flow of the dialysis solution towards the peritoneal cavity.

These and other features of the invention appear from the following description referred to a preferred embodiment of the automatic apparatus for peritoneal dialysis which is illustrated, for exemplifying but non limiting purpose, in the accompanying figures, in which:

Figure 1 shows the principle scheme of operation of the apparatus for peritoneal dialysis of the invention according to a preferred embodiment;

Figure 2 is a partial cross section of the apparatus of Figure 1;

Figure 3 shows a detail of Figure 1;

Figures 4 and 5 show the operating scheme of another embodiment of the invention; and

Figure 6 shows the pressure-operating time chart of the apparatus of the invention.

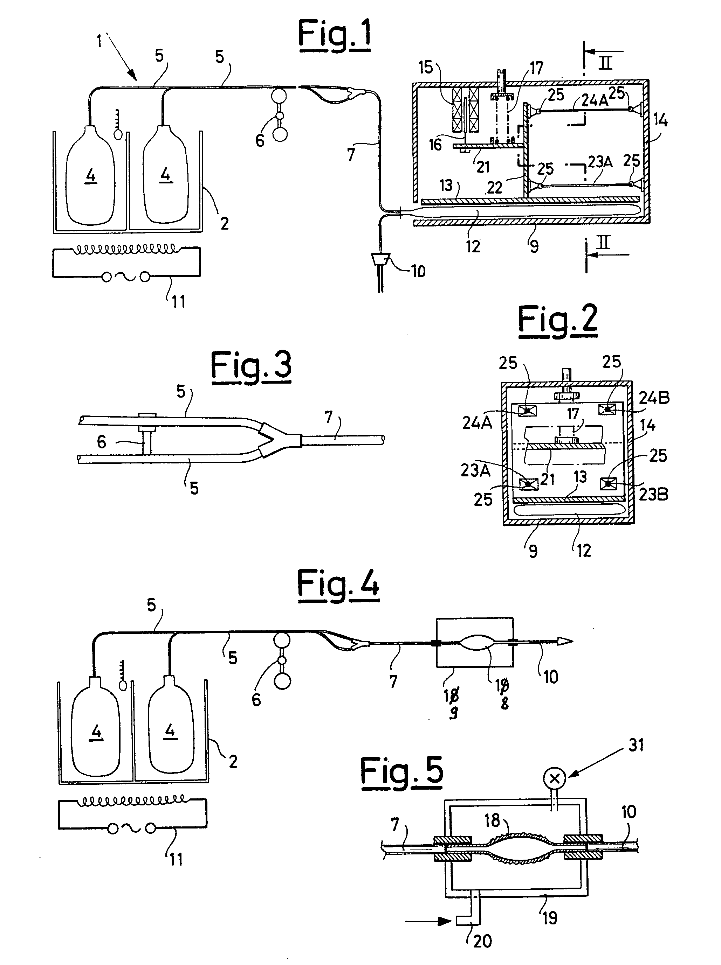

Referring to Figure 1, the apparatus is indicated on the whole by reference 1 and essentially comprises:

a shaped body 2 for containing the auxiliary parts and the electrical components;

a pair of bags 4 for feeding and collecting the dialysis solution, each one being provided with an independent tubular duct 5 and being thermostatized, particularly heated by means of heating resistances 11;

a pump 6 with double circulation direction, of known type, which is mounted at the tubular ducts 5;

a tubular duct 7, in which the tubular ducts 5 merge by means of a suitable fitting;

a frame 9 for supporting and containing sensing means for the control of the pump 6;

a fitting 10 of known type and adapted for the connection to a catheter (not shown) already positioned in the peritoneal cavity, particularly of the type permitting the liquid flow towards and from the peritoneal cavity.

The apparatus of Figure 1 moreover comprises the several control systems (valves, electrical components), the use of which is since long time matter per se known and which are omitted for sake of clarity.

It is worth to mention that the pump 6 is of the peristaltic type with double pumping pipe for a suitable distribution of the feeding and emptying flow with respect to the two bags and is driven by a reversible electrical motor which makes the pump bidirectional, with possibility of flow rate adjustment by means of adjustment of the motor speed.

Within the frame 9 the following components are housed:

a bag 12 of flexible material adapted to receive the dialysis solution from the duct 7 and connected to the fitting 10;

a horizontal plane 13 which is vertically slidable and abutting by gravity onto the bag 12;

a spiral spring 17 which is calibrated to press onto a bracket 21, which is rigidly connected to the vertical movable plate 22 which in turn is part of an articulated parallelogram mechanism and comprising the fixed wall 14 of the frame 9 and four tie-rods, which are two by two parallel to each other, 23 (A, B) and 24 (A, B) and which are hinged at their ends (as indicated by 25) respectively to the plate 22 and to the wall 14.

A displacement of the movable plane 13 as caused by a dilatation of the bag 12 causes an upwardly directed vertical displacement of the plate 22, parallel to itself and the deformation of the articulated parallelograph (14, 22, 23, 24) around the hinges 25. The displacement of the plate 22 causes the bracket 21 to be raised against the action of the spring 17.

The tie rods 23 and 24 are preferably elastic, namely capable of undergoing a bending with respect to their main axis, so as to compensate displacements of the plane 13 in a manner which is not parallel to itself, namely when the plane 13 does not remain horizontal.

a linear displacement transducer (LVDT), of a per se known type, connected to driving motor of the pump 6, having an electromagnetic coil 15 fixed to the frame 9 of the apparatus and a rod or armature 16, cooperating with the coil and rigidly connected to the bracket 21.

According to the shown embodiment the bags 4 contain at the very beginning an amount in excess with respect to that of presumable loading of the peritoneal cavity, the bag 12 is empty and squashed onto the bottom of the frame 9 by effect of the spring 17 and the peritoneal cavity is empty of dialitic solution (point 0 of the chart of Figure 5).

By actuating the pump 6 the dialitic solution is pumped into the circuit comprising the duct 5, the duct 7 and the bag 12, the fitting 10 and lastly, through the catheter, the peritoneal cavity (not shown).

The action of the pump thus generates in the delivery circuit a pressure $P_A$ causing the dilatation of bag 12.

The increase of volume of the bag 12 causes the plane 13 to be raised and displaced upwardly by remaining horizontal.

This displacement, through the above disclosed articulated parallelogram mechanism, determines the compression of the spring until the latter reacts with a force $R_A$ which equilibrates the pressure $P_A$ established within the bag 12.

This displacement, which as already stated may be repeated in time, is detected by the transducer 15, 16.

During the time in which the pressure $P_A$ is attained, little liquid enters the peritoneal cavity, owing to the high resistance raised by the connecting pipe to the patient and by the catheter (the pressure drop, with a flow rate of 200 ml/min and with typical resistances to the flow, being about 400 mm $H_2O$).

Upon the pressure $P_A$ is attained and the pump is stopped, the spring 17 shall tend to press the plane 13 with a force $R_A$, whereby the bag 12 is emptied for the filling of the patient's peritoneal cavity.

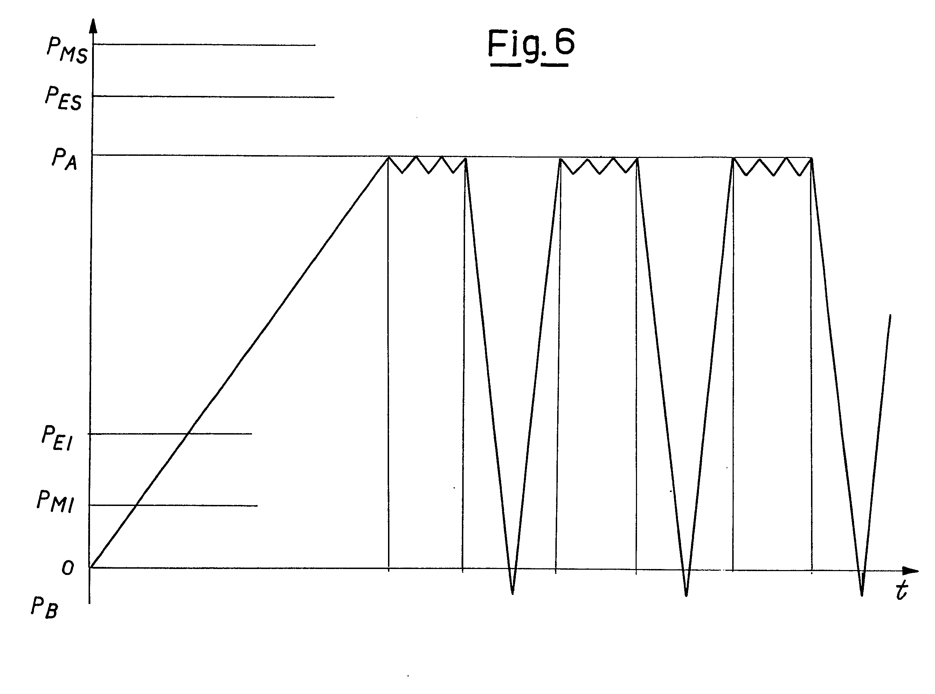

A period of pressure settling does thus begin, since, the pressure decreases (by a value which can be introduced to determine the hysteresis of the system), and the pump will be restarted, so as to fill again the bag 12. Thus, the above behaviour will be repeated until the pressure within the peritoneal cavity shall be statically equal to $P_A$ (which is typically selected at 200 mm $H_2O$), as shown in Fig. 6 by the zigzag part of plot. Consequently, independently from the resistance of the pipe and of the catheter, which may vary also by a great value, for instance owing to fibrine deposition, the loading of the peritoneal cavity will terminate when the pressure of the peritoneal cavity shall be equal to the selected one $P_A$. The time necessary for the disappearance of the "dynamic" component of the pressure as measured by the transducer, i.e. the time required to the termination of the series of pressure adjustments, is typically of about 5 minutes, but may vary from a dialitic treatment to another one and is also dependent as already stated on the various hydraulic resistances, both distributed and localized exhibited by the flow circuit.

Upon the pressure $P_A$ is definitely attained in the peritoneal cavity, the pump is inverted, whereby the peritoneal cavity is emptied.

The pump feeds back to the bags 4 a predetermined amount of dialitic solution which is removed from the peritoneal cavity, and is preferably lesser than the initially fed amount.

The transfer of such a liquid amount from the peritoneal cavity to the bags 4 causes the pressure in the bag 12 to be reduced from $P_A$ zero or even to a negative pressure after the aforesaid settlement periods; it in turn determines a negative variation of the bag volume, with consequent lowering of the plane 13.

A timing device causes the rotation of the pump 4 to be inverted. In this way there is made possible to detect pressures and their variations comprised between 100 and 600 mm $H_2O$. It is to be noticed that the spring 17 is calibrated so that it generates a force such as to prevent the plane 13 from being raised owing to possible abrupt variations of pressure in the bag 12, as caused for instance by fits of coughing of the patient and by other factors extraneous to the transfer of dialysis solution to and from the peritoneal cavity.

It is also ensured that accidental pressure increases do not prevent the desired pressure $P_A$ from being attained; on the contrary the transducer 15, 16 would actuate the pump, in the filling phase, so as to start the sucking step with removal from the peritoneal cavity of an amount of dialysis solution greater than the predetermined one.

In the case in which a low pressure drop exist in the circuit (such as a few mm $H_2O$), it is also possible to obtain a pressure emptying, i.e. until a minimum pressure is achieved. Of course this situation depends on the availability of a suitable catheter. The inversion of the pump causes the pressure to be again increased from $P_B$ to $P_A$, correspondingly to the volume increase of the bag 12, up to previous volume, sensed by the transducer 15, 16, to which the transfer of the desired amount of dialysis solution from the bags 4 to the peritoneal cavity corresponds. The complete dialitic treatment extends over about 8 hours.

Warning sensing means (not shown) are moreover provided in the frame 9 for sensing a minimum and maximum level of plane 13, which are adjusted so as to be actuated in case of operating defect of the transducer 15, 16 in case of damage of the bag 12 and in case of loss of calibration of the spring 17. The circuit is also provided with sensing means of negative pressure.

These warning thresholds are theoretically double (namely upper and lower) since one pair is provided as safety for the transducer 15, 16 and the other one for the displacement of the plate 13.

The related thresholds in Figure 6 are indicated by pressure values which are corresponding and adjustably provided.

$P_{ES}$ indicates the predetermined upper threshold value for the transducer 15, 16; $P_{MS}$ indicates the predetermined upper threshold value for the plate 13.

Obviously only upper threshold limits are described, since in this embodiment the emptying phase is controlled by timing means.

In the embodiment of Figures 4 and 5, the bag 12 has been substituted for by a dilatable body or bellows housed within a chamber 19 filled with a pressure fluid, for instance air, through the fitting 20. The behaviour of the bellows 18, for instance of elastic synthetic material, is like that of the bag 12.

In this embodiment, the volume increase of bellows 18 is opposed by the air pressure within the chamber 19. The increase and the diminution of volume of the bellows 18 is turned into an increase and respectively diminution of pressure in the chamber 19, which are sensed by a pressure transducer 31 controlling the motion of the pump 6 according to the pressure limit value $P_A$ as attained in the peritoneal cavity.

The residual air pressure in the chamber 19, within the bellows 18 upon the peritoneal cavity has been emptied, is such as to compensate abrupt pressure variations.

Of course it is possible to carry out further variations and modifications which, however, if based on the general concept of automatically controlling the circulation of the dialysis solution on the basis of measurements of volume of a dilatable body or element inserted in the dialysis circuit, fall within the protection scope of the above described invention.

Otherwise stated, the dilatable compensating bag 12 operates as a flow element of the dialysis solution, as a tank for collection and the compensation of abrupt variations of pressure taking place downstream of the same bag, and as a mechanical signalling member of the solution volume fed to the peritoneal cavity. At the same time the maximum safety conditions of operation of the dialysis apparatus are maintained.

## Claims

1. Automatic apparatus for peritoneal dialysis comprising:

at least one bag (4) for the feeding and collecting of the dialysis solution;

a connecting duct (5) between each bag and the peritoneal cavity of the patient, and

a reversible rotating pump (6) to carry out the circulation of the dialysis solution in said connecting duct towards and away from the peritoneal cavity, characterized by comprising:

first dilatable compensating means (12) having a controlled dilatation and in liquid communication with said peritoneal cavity, said first means (12) with controlled dilatation are connected in series to the length of said connecting duct (5) between the pump (6) and the patients peritoneal cavity and are capable of compensating abrupt pressure variations in said duct, the dilatation of said first means being caused by the pressure of the dialysis solution circulating within the same length of duct, and

second means (15, 16, 21, 22, 13) associated to said first means (12) being capable of measuring the dilatation of said first means and correspondingly controlling the pump operation, at least in the phase of filling of the peritoneal cavity with the dialysis solution.

2. Apparatus of claim 1, characterized in that said first means (12) with controlled dilatation are adapted such that no dilatation occurs when the pressure of the dialysis solution in the said length of duct attains a lower limit value.

3. Apparatus of claim 2, characterized in that said lower limit value corresponds to a predetermined emptying condition of the peritoneal cavity.

4. Apparatus of claim 1, characterized in that said first means (12) comprise a bag of pressure dilatatable material serially connected in said length of duct and a spring (17) opposing the dilatation of said bag acting between a dilatable wall of the bag and a fixed supporting part of the dialysis apparatus.

5. Apparatus of claim 1, characterized in that said second dilation measuring means comprise a displacement linear transducer consisting of an electromagnetic coil (15) secured to a fixed supporting part of the apparatus and a movable armature (16) cooperating with the said coil so as to vary the electromagnetic flux linked with the coil, the armature (16) being fastened to at least a freely movable wall of said first dilatable means.

6. Apparatus of claims 1 and 4, characterized in that said second measuring means comprise a plane (13) abutting by gravity against the upper surface of said bag of dilatable material, said plane being subjected to the action of said contrasting spring (17).

7. Apparatus of claims 5 and 6, characterized in that said movable armature (16) is rigidly connected to said movable plane (13).

8. Apparatus of claim 6, characterized in that said movable plane (13) is connected to a vertical plate (22) carrying a horizontal bracket (21) against which said contrasting spring (17) acts.

9. Apparatus of claim 8, characterized in that said vertical plate (22) is fastened to the fixed supporting part of the apparatus through an articulated parallelogram.

10. Apparatus of claim 9, characterized in that said vertical plate (22) and a vertical wall (14) of said apparatus form two opposite sides of said parallelogram, the other two sides being formed by tie rods (23, 24) hinged at their ends to said vertical plate and to said vertical wall.

11. Apparatus of claim 10, characterized in that said rods (23, 24) are flexible with respect to their main axis.

12. Apparatus of claims 7 and 8 characterized in that said movable armature (16) is rigidly connected to said bracket (21).

## Patentansprüche

1. Automatische Vorrichtung zur Peritonealdialyse, mit

mindestens einem Behälter (4) zur Zufuhr und Sammlung der Dialyselösung;

einer Verbindungsleitung (5) zwischen jedem Behälter und der Peritonealhöhle des Patienten, und

einer umsteuerbaren Rotationspumpe (6) zur Umwälzung der Dialyselösung in der Verbindungsleitung zur und von der Peritonealhöhle; gekennzeichnet durch

erste aufweitbare Kompensationsmittel (12) mit geregelter Ausdehnbarkeit und in Flüssigkeitsverbindung mit der Peritonealhöhle, wobei diese ersten Mittel (12) mit geregelter Ausdehbarkeit in Reihe mit dem Stück der Verbindungsleitung (5) zwischen der Pumpe (6) und der Peritonealhöhle des Patienten verbunden und zum Ausgleich abrupter Druckänderungen in der genannten Leitung befähigt sind, und wobei die Ausdehnung der ersten Mittel durch den Druck der Dialyselösung bewirkt wird, die in dem gleichen Leitungsstück strömt, und

zweite, den ersten Mitteln (12) zugeordnete Mittel (15, 16, 21, 22, 13), die dazu befähigt sind, die Ausdehnung der ersten Mittel zu messen und dementsprechend den Betrieb der Pumpe zu steuern, zumindest während des Füllens der Peritonealhöhle mit Dialyselösung.

2. Vorrichtung nach Anspruch 1, dadurch gekennzeichnet, dass die ersten Mittel (12) mit geregelter Ausdehnbarkeit derart eingerichtet sind, dass keine Ausdehnung stattfindet, wenn der Druck der Dialyselösung im genannten Leitungsstück einen unteren Grenzwert erreicht.

3. Vorrichtung nach Anspruch 2, dadurch gekennzeichnet, dass der untere Grenzwert einer vorgegebenen Entleerungsbedingung der Peritonealhöhle entspricht.

4. Vorrichtung nach Anspruch 1, dadurch gekennzeichnet, dass die ersten Mittel (12) einen Behälter aufweisen, der aus einem unter Druck dehnbaren Material besteht und der in Reihe mit dem genannten Leitungsstück verbunden ist, sowie eine Feder (17), die einer Ausdehnung des Behälters zwischen einer dehnbaren Wandung des Behälters und einem festen tragenden Teil der Dialysevorrichtung entgegenwirkt.

5. Vorrichtung nach Anspruch 1, dadurch gekennzeichnet, dass die zweiten Mittel zur Messung der Ausdehnung einen linearen Verschiebungsgeber aufweisen, der aus einer elektromagnetischen Spule (15), die an einem ortsfesten Trägerteil der Vorrichtung angebracht ist, und einem beweglichen Anker (16) besteht, der mit der Spule derart zusammenwirkt, dass der mit der Spule gekoppelte elektromagnetische Fluss geändert wird, wobei der Anker (16) an mindestens einer frei beweglichen Wandung der genannten ersten ausdehnbaren Mittel angebracht ist.

6. Vorrichtung nach Anspruch 1 und 4, dadurch gekennzeichnet, dass die zweiten Mittel zur Messung eine Ebene (13) aufweisen, die durch Schwerkraft auf der oberen Fläche des Behälters aus dehnbarem Material aufliegen und die der Wirkung der genannten Gegenfeder (17) ausgesetzt ist.

7. Vorrichtung nach Anspruch 5 und 6, dadurch gekennzeichnet, dass der bewegliche Anker (16) starr mit der beweglichen Ebene (13) verbunden ist.

8. Vorrichtung nach Anspruch 6, dadurch gekennzeichnet, dass die bewegliche Ebene (13) mit einer vertikalen Platte (22) verbunden ist, an der ein horizontaler Träger (21) befestigt ist, gegen den die Gegenfeder (7) wirkt.

9. Vorrichtung nach Anspruch 8, dadurch gekennzeichnet, dass die vertikale Platte (22) mit dem festen Trägerteil der Vorrichtung über ein Gelenkparallelogramm verbunden ist.

10. Vorrichtung nach Anspruch 9, dadurch gekennzeichnet, dass die vertikale Platte (22) und eine vertikale Wandung (14) der Vorrichtung zwei Gegenseiten des Parallelogramms bilden, und dass die beiden anderen Seiten durch Verbindungsstäbe (23, 24) gebildet werden, die an ihren Enden gelenkig mit der vertikalen Platte und der vertikalen Wandung verbunden sind.

11. Vorrichtung nach Anspruch 10, dadurch gekennzeichnet, dass die Stäbe (23, 24) bezüglich ihrer Hauptachse biegsam sind.

12. Vorrichtung nach Anspruch 7 und 8, dadurch gekennzeichnet, dass der bewegliche Anker (16) starr mit dem genannten Träger (21) verbunden ist.

## Revendications

1. Appareil automatique pour la dialyse péritonéale, comportant

au moins un récipient (4) pour délivrer et recevoir la solution de dialyse;

une conduite de raccordement (5) entre chaque récipient et la cavité péritonéale du patient, et

une pompe rotative réversible (6) pour effectuer la circulation de la solution de dialyse dans la conduite de raccordement vers la cavité péritonéale et en retour, caractérisé en ce qu'il comprend:

des premiers moyens de compensation dilatables (12) ayant une dilatation contrôlée et étant en communication de liquide avec la cavité péritonéale, les premiers moyens (12) à dilatation contrôlée étant reliés en série à la section de la conduite de raccordement (5) entre la pompe (6) et la cavité péritonéale du patient et étant capables de compenser des variation abruptes de pression dans la dite conduite, la dilatation des dits premiers moyens étant causée par la pression de la solution de dialyse qui circule dans la même section de la conduite, et

des seconds moyens (15, 16, 21, 22, 13) associés aux dits premiers moyens (12), capables de mesurer la dilatation des premiers moyens et de contrôler conformément l'opération de la pompe au moins pendant le remplissage de la cavité péritonéale avec la solution de dialyse.

2. Appareil selon la revendication 1, caractérisé en ce que les dits premiers moyens (12) à dilatation contrôlée sont agencés tels qu'il n'y a pas de dilatation lorsque la pression de la solution de dialyse dans la dite section de conduite atteint une valeur limite inférieure.

3. Appareil selon la revendication 2, caractérisé en ce que la valeur limite inférieure correspond à une condition d'évacuation prédéterminée de la cavité péritonéale.

4. Appareil selon la revendication 1, caractérisé en ce que les premiers moyens (12) comportent un récipient en un matériau dilatable sous pression, relié en série à la dite section de conduite, et un ressort (17) s'opposant à la dilatation du récipient, agissant entre une paroi dilatable du récipient et une partie de support fixe de l'appareil de dialyse.

5. Appareil selon la revendication 1, caractérisé en ce que les seconds moyens de mesure de dilatation comportent un transducteur linéaire de déplacement contenant une bobine électromagnétique (15), attachée à une partie de support fixe de l'appareil, et une armature mobile (16) coopérant avec la bobine pour faire varier le flux électromagnétique associé à la bobine, l'armature (16) étant fixée à au moins une paroi librement déplaçable des premiers moyens dilatables.

6. Appareil selon les revendications 1 et 4, caractérisé en ce que les seconds moyens de mesure comprennent un plan (13) s'appuyant par gravité sur la surface supérieure du récipient en matériau dilatable, le dit plan étant soumis à l'action du dit ressort de charge (17).

7. Appareil selon les revendications 5 et 6, caractérisé en ce que l'armature mobile (16) est rigidement reliée au dit plan mobile (13).

8. Appareil selon la revendication 6, caractérisé en ce que le dit plan mobile (13) est relié à une plaque verticale (22) supportant une traverse horizontale (21) contre laquelle agit le ressort de charge (17).

9. Appareil selon la revendication 8, caractérisé en ce que la plaque verticale (22) est fixée à la partie de support fixe de l'appareil par un parallèlogramme articulé.

10. Appareil selon la revendication 9, caractérisé en ce que la plaque verticale (22) et une paroi verticale (14) de l'appareil forment deux côtés opposés du dit parallèlogramme, les deux autres côtés étant formés par des tiges de raccord (23, 24) articulées à leurs extrémités à la plaque verticale et à la paroi verticale.

11. Appareil selon la revendication 10, caractérisé en ce que les tiges (23, 24) sont flexibles par rapport à leurs axes principaux.

12. Appareil selon les revendications 7 et 8, caractérisé en ce que l'armature mobile (16) est rigidement fixée à la traverse (21).

# Fig.1

# Fig.2

# Fig.3

# Fig.4

# Fig.5

Fig.6